(19) Europäisches Patentamt / European Patent Office / Office européen des brevets

(11) **EP 2 235 474 B1**

(12) **FASCICULE DE BREVET EUROPEEN**

(45) Date de publication et mention
de la délivrance du brevet:
**21.10.2015 Bulletin 2015/43**

(21) Numéro de dépôt: **09706602.1**

(22) Date de dépôt: **21.01.2009**

(51) Int Cl.:
*G01J 5/02* (2006.01)  *G01N 33/20* (2006.01)
*G01J 5/08* (2006.01)  *G01J 5/00* (2006.01)
*G01N 25/72* (2006.01)  *G01B 21/08* (2006.01)

(86) Numéro de dépôt international:
**PCT/EP2009/050617**

(87) Numéro de publication internationale:
**WO 2009/095338 (06.08.2009 Gazette 2009/32)**

(54) **CONTROLE D'UN TRAITEMENT DE DECAPAGE**

**STEUERUNG EINES ÄTZVERFAHRENS**

**ETCHING PROCESS CONTROL**

(84) Etats contractants désignés:
**AT BE BG CH CY CZ DE DK EE ES FI FR GB GR
HR HU IE IS IT LI LT LU LV MC MK MT NL NO PL
PT RO SE SI SK TR**

(30) Priorité: **31.01.2008 BE 200800061**

(43) Date de publication de la demande:
**06.10.2010 Bulletin 2010/40**

(73) Titulaire: **Centre De Recherches Metallurgiques
ASBL - Centrum
Voor Research In De Metallurgie vzw
1000 Bruxelles (BE)**

(72) Inventeurs:
• **CRAHAY, Jean
B-4970 Francorchamps (BE)**

• **MOREAS, Geneviève
B-4520 Moha (Wanze) (BE)**

(74) Mandataire: **Pronovem
Office Van Malderen
Parc d'affaires Zénobe Gramme- bâtiment K
Square des Conduites d'Eau 1-2
4020 Liège (BE)**

(56) Documents cités:
**EP-A- 1 521 056  JP-A- 11 325 839**

• **CZICHOS, H.: "HÜTTE DIE GRUNDLAGEN DER
INGENIEUR-WISSENSCHAFTEN" 2000,
SPRINGER , BERLIN , XP002519226 page D54 -
page D55**

**Description**

**Objet de l'invention**

La production de bandes d'acier comprend -une série d'étapes successives :

- une fois élaboré, l'acier liquide est coulé, le plus souvent en continu sous forme de brames de quelques centimètres d'épaisseur ;
- il est laminé à haute température pour profiter d'une grande ductilité dans ce domaine ; on crée ainsi une bande de métal généralement de quelques millimètres d'épaisseur qui est bobinée et refroidie jusqu'à température ambiante ;
- le décapage enlève la couche d'oxyde et la bande d'acier est soit découpée en tôle pour être utilisée, soit laminée à froid vers des épaisseurs descendant jusqu'à 0,1 mm pour les applications les plus minces dans le domaine de l'emballage.

L'étape de décapage précitée fait l'objet de la présente invention. Elle concerne plus particulièrement les aciers dits « au carbone » ainsi que les aciers plus fortement alliés comme les aciers inoxydables riches en chrome et nickel entre autres ou comme les aciers à haute teneur en silicium destinés aux applications dans l'industrie électrique.
Un autre objet de la présente invention est de pouvoir détecter la présence d'oxyde résiduel sur la bande au laminoir à chaud, après décalaminage.
Enfin, un autre domaine d'application de l'invention est la détection d'oxydes sur les cylindres de laminoir.

**Arrière-plan technologique et position du problème**

Il est connu qu'une couche d'oxyde est créée lors du laminage à chaud de bandes d'acier, celui-ci ayant lieu à des températures allant typiquement de 900°C à 1200°C. Le laminage à chaud effectué généralement sous air ambiant, il se produit ainsi une oxydation très forte de la surface métallique. La couche d'oxyde créée comprend évidemment un oxyde de fer mais aussi des oxydes des éléments d'alliage tels que Mn, Cr, Al, Si, etc.
En fin de laminage à chaud, la bande d'acier est refroidie par projection d'eau jusqu'à des températures allant de 500°C à 750°C, après quoi elle est bobinée.
Les bobines massives (10 à 20 tonnes typiquement) vont mettre plusieurs jours à se refroidir et pendant ce temps l'oxyde qui se trouve à la surface va continuer à évoluer. Cette évolution se marque sous deux aspects :

- nature de l'oxyde : il existe différents oxydes de fer, essentiellement la wustite $FeO$, la magnétite $Fe_3O_4$ et l'hématite $Fe_2O_3$. Les différents oxydes de fer ont des domaines de température de stabilité thermique qui leurs sont propres. Lors du refroidissement des bobines, l'oxyde de fer se transforme généralement en un mélange d'oxydes. La composition et la structure de ce mélange dépend de la composition chimique de l'acier, des conditions de laminage à chaud, de la température de bobinage, de la masse de la bobine et de la vitesse de refroidissement. En effet, il est possible entre autres d'influencer la composition du mélange d'oxydes en refroidissant les bobines de manière énergique ;
- épaisseur de la couche d'oxyde : les bobines sont enroulées en spires serrées mais ne constituent pas pour autant des domaines étanches. Lors du refroidissement, l'air pénètre entre les spires et l'oxygène peut encore accentuer l'oxydation. Etant donné que les spires demeurent assez serrées, la pénétration de l'air est principalement marquée aux extrémités de la bobine : tranches latérales, spires extérieures et spires intérieures. Dans toutes ces zones, on observe une oxydation plus forte.

En conclusion, la couche d'oxyde présente à la surface des bandes lorsque les bobines ont été refroidies est fortement variable :

- la composition de la couche d'oxyde, c'est-à-dire en oxydes de fer et autres éléments, dépend de la composition chimique de l'acier et des conditions de laminage à chaud ;
- la nature des oxydes de fer qui sont majoritaires dépend des conditions de laminage mais aussi de la température de bobinage et des conditions de refroidissement ;
- en outre, la distribution des propriétés des oxydes est très hétérogène : les pourtours de la bande (qui a typiquement une largeur de 1~2 m et une longueur de plusieurs centaines de mètres) sont plus fortement oxydés ;
- l'épaisseur de la couche d'oxyde est typiquement de 3 à 10 $\mu$m.

*Le décapage*

Le décapage est un procédé chimique qui permet d'éliminer des couches d'oxyde de la surface de métaux. On obtient ainsi une surface plus propre, d'aspect plus attrayant et plus apte à subir des traitements ultérieurs. En particulier, lors d'un laminage à température ambiante, l'oxyde qui resterait sur la bande risquerait d'être pulvérisé et de polluer les cylindres de laminoir, entraînant une perte de qualité importante.

Le décapage a lieu en mettant la surface de la bande d'acier en contact avec une solution d'acide chaude.

Les bains utilisés pour le décapage sont couramment des solutions aqueuses d'acides chlorhydrique ou sulfurique dans le cas des aciers « au carbone ». Pour les aciers plus fortement alliés, on a recours à des solutions plus agressives contenant aussi les acides nitrique ou fluorhydrique.

S'il existe encore des installations de décapage qui travaillent en aller-retour (ligne dites « push-pull »), la plupart des installations de décapage fonctionnent en continu. A l'entrée de la ligne, les bobines successives sont assemblées et c'est une bande continue qui traverse la station de décapage. Des accumulateurs en amont et en aval de la portion de décapage permettent de gérer les variations de vitesse des différentes sections. En outre, une tendance de plus en plus marquée est de coupler la décaperie et le laminage qui la suit dans une même ligne : on fait ainsi l'économie de bobinage et débobinage intermédiaires.

La mise en contact de la bande à décaper et de la solution acide peut se faire de différentes manières : la plus ancienne consistait à faire défiler la bande en la trempant dans une succession de bains chacun long de plusieurs dizaines de mètres. Dans des conceptions plus récentes, la solution acide est projetée sur la bande au moyen de pompes et de gicleurs. Cette disposition permet d'accélérer le décapage par des effets de renouvellement très rapide de l'acide au contact de la bande. En outre elle permet un contrôle plus précis du décapage et des situations transitoires, comme l'arrêt de la ligne pour raison d'assemblage difficile de bandes par exemple. De manière générale, la partie traitement d'une ligne de décapage actuelle comprend :

- une série de bacs de décapage dans lesquels la bande d'acier est en contact avec la solution acide, soit par immersion, soit par projection ;
- une section de rinçage destinée à éliminer tout résidu de solution acide à la surface de la bande qui vient d'être décapée. En effet, toute pollution superficielle en solution acide ou en sels (chlorures de fer, par exemple) provoquerait un défaut de surface visible et/ou un risque de corrosion en cours de stockage ou de transport ;
- un séchage final.

*Le contrôle du décapage*

Un décapage correctement effectué doit satisfaire aux spécifications suivantes :

- enlever la totalité de l'oxyde qui se trouve à la surface de la bande au sortir du laminage à chaud. Si tout l'oxyde n'est pas enlevé, on parle de « sous-décapage » ;
- ne pas trop attaquer le fer qui constitue la bande d'acier. En effet, les solutions acides utilisées pour enlever l'oxyde sont aussi capables de réagir avec le fer et de l'attaquer, c'est ce qu'on appelle le « sur-décapage ». Le respect de cette exigence d'éviter le sur-décapage est facilité par l'ajout de substances chimiques (dites inhibiteurs) qui se fixent non sur l'oxyde mais bien sur le fer et le protègent de l'acide ;
- optimiser les temps de traitement et partant la productivité de la ligne.

Pour atteindre ces objectifs de qualité, la bande sortant de la ligne est habituellement inspectée par des personnes qui en observent la surface pour repérer tout défaut. Comme dans d'autres domaines, on cherche à remplacer cette inspection visuelle par des systèmes automatiques ; ils se justifient par une grande régularité de fonctionnement et une possibilité de détecter des défauts même de petite taille.

**Etat de la technique en contrôle du décapage**

Le problème du contrôle du processus de décapage peut s'énoncer comme celui d'enlever la couche d'oxyde de manière totale mais sans excès, alors que :

- les propriétés de cette couche d'oxyde varient d'une bobine à l'autre ;
- les propriétés de l'oxyde sont fortement hétérogènes, le centre de la bande étant nettement plus facile à décaper que les rives ;
- la composition chimique des bains varie au cours du temps suite aux réactions chimiques dont ces bains sont le siège (appauvrissement en acide et enrichissement en chlorures de fer dans le cas du décapage à l'acide chlorhydrique).

Le paramètre de processus le plus facilement utilisable pour le contrôle du décapage est la vitesse de ligne. Elle est rapidement modulable et a un effet direct sur l'état de décapage.

En conséquence, plusieurs stratégies ont été successivement mises en place pour assurer un meilleur contrôle du décapage. Ces stratégies peuvent être décrites comme suit et développées ci-après :

- meilleure maîtrise de la composition chimique des bains de décapage ;
- adaptation des paramètres de traitement en fonction des propriétés présumées de l'oxyde ;
- adaptation des paramètres de traitement en fonction de paramètres d'oxyde mesurés à différents endroits de la ligne ;
- détection automatique de défauts par inspection de toute la surface de la bande par caméra rapide et traitement d'images.

*Meilleure maîtrise de la composition des bains de décapage*

Celle-ci a été obtenue dans un premier temps par des consignes de réapprovisionnement en acide des bacs.

Ensuite, on a procédé à des mesures systématiques des concentrations en acide et en chlorure (ou sulfate) de fer. Actuellement vient sur le marché une nouvelle génération de systèmes d'analyse pouvant fonctionner de manière automatique.

*Adaptation en fonction des propriétés présumées de l'oxyde*

L'influence de la nature de l'acier ainsi que celles de la température de bobinage et du mode de refroidissement sont relativement bien connues.

Des stratégies de contrôle de la vitesse de ligne ou de la concentration en acide ont été mises en avant, basées sur la connaissance de la composition chimique et des conditions de laminage à chaud des différentes bobines.

C'est par exemple le cas dans la demande de brevet JP-A-2003 231981 déposée par Nippon Steel et qui propose d'adapter la vitesse de la ligne en fonction du type d'acier, des conditions de laminage à chaud, du mode de préparation de surface avant décapage ainsi que des paramètres de la ligne de décapage.

*Adaptation en fonctions des paramètres d'oxyde mesurés à*

*différents endroits*

L'oxyde qui doit être enlevé est mesuré soit à l'entrée de la ligne, soit en cours de traitement. Les paramètres de traitement sont adaptés en fonction des propriétés détectées pour cet oxyde. C'est par exemple le cas dans les demandes :

- JP-A-11 325839, déposée par Kawazaki Steel et qui propose d'effectuer une mesure précise de l'épaisseur de la couche d'oxyde à l'aide de pyromètres infrarouges à deux longueurs d'onde ;
- JP-A-11 189885, déposée par Kawazaki Steel et qui propose de réguler les paramètres de décapage en fonction de l'épaisseur de la couche d'oxyde entrante ;
- JP-A-10 306391, déposée par Hitachi et qui propose d'optimiser les conditions de traitement sur base de capteurs d'état de bande et d'état de ligne ainsi que sur base d'un algorithme de contrôle ;
- DE-A-103 32 693, déposée par SMS Demag et qui propose de mesurer les propriétés de l'oxyde en deux points et d'adapter la pression d'une section d'arrosage. La mesure des propriétés d'oxyde n'est pas détaillée mais il peut s'agir de la présence d'oxyde demeurant à la surface ;
- EP-A-1 162 287, déposée par SMS Demag et qui propose de mesurer les propriétés de l'oxyde, en termes de présence et structure, en plusieurs points de la ligne de décapage (deux points de mesure par exemple) et d'adapter les paramètres de la ligne de décapage. La mesure des propriétés de la couche d'oxyde est effectuée par des pyromètres infrarouges dont le gain est réglable et qui peuvent détecter les propriétés des différents oxydes sur base de leur émissivité.

*Détection de défauts par inspection automatique de toute la surface de la bande et traitement d'images*

Cette dernière génération d'appareillages inspecte toute la surface de la bande à l'aide de caméras opérant dans le domaine visible. Un traitement d'image détecte les hétérogénéités et les interprète en termes de défauts. Ces défauts sont généralement produits en amont mais ne sont révélés qu'après décapage.

## Buts de l'invention

La présente invention vise à fournir une solution pour contrôler la présence d'oxydes sur une bande d'acier laminée à chaud, qui s'affranchisse des inconvénients inhérents à l'état de la technique.

Plus précisément, dans ce contexte, l'invention vise à fournir une technique de détection automatique, en ligne et en continu du sous-décapage qui soit à la fois fiable et très sensible.

## Principaux éléments caractéristiques de l'invention

Un premier objet de la présente invention selon la revendication 1 se rapporte à un procédé de détection d'une couche d'oxyde sur une bande métallique en mouvement dans une installation de fabrication en continu, dans lequel on observe la bande en mouvement à l'aide d'un système de détection sans contact basé sur la mesure du rayonnement thermique émis par ladite bande, caractérisé en ce qu'on mesure la température apparente de la bande en une pluralité de zones, c'est-à-dire que ladite mesure est effectuée en omettant volontairement d'appliquer une correction d'émissivité et en ce qu'on identifie une zone recouverte d'oxyde par une valeur de température apparente sensiblement supérieure à celle d'une ou plusieurs autres zones dépourvues d'oxyde.

Selon des formes d'exécution préférées, le procédé de l'invention comporte en outre, en combinaison, une ou plusieurs des caractéristiques suivantes :

- fixer le seuil de détection à un écart de température apparente d'au moins 5°C, de préférence au moins 10°C ;
- observer la bande en un endroit où elle est sèche ;
- observer la bande en un endroit où sa température est relativement homogène ;
- observer la bande en un endroit où sa température est supérieure à 40°C et de préférence à 50°C ;
- utiliser une moyenne mobile des températures apparentes pour appliquer le critère de détection et identifier des zones de dépassement de seuil ;
- observer la bande point par point à l'aide d'un pyromètre ;
- observer toute la surface de la bande en utilisant une caméra thermique ;
- utiliser un système de mesure selon une ligne ou une matrice à deux dimensions, de préférence à balayage rapide ;
- acquérir plus de 50 et de préférence 100 points-image par ligne, de préférence sur la largeur de la bande ;
- utiliser une fréquence de balayage supérieure à 100Hz, de préférence supérieure à 500Hz ;
- scruter des zones de l'ordre de 1 cm$^2$ ;
- reconstituer une image continue en juxtaposant les lignes par traitement d'image ;
- coupler la détection thermique avec une détection vidéo fonctionnant essentiellement dans la même zone de scrutation ;
- activer le système vidéo lorsque la température apparente dépasse un certain seuil ;
- utiliser le signal vidéo comme alarme et indication de zone à défaut pour un opérateur, comme enregistrement pour le contrôle qualité ou en vue d'un traitement ultérieur.

Le procédé selon l'invention peut avantageusement être appliqué pour :

- contrôler le décapage de bandes d'acier laminées à chaud, en détectant les zones d'oxyde résiduel qui subsistent après décapage ;
- détecter un oxyde résiduel sur bande d'acier laminée à chaud, après décalaminage ;
- détecter la présence d'oxyde sur des cylindres de laminage.

Un second objet de la présente invention selon la revendication 19 se rapporte à une installation de décapage intégrée dans une ligne de production de bandes d'aciers, de préférence laminées à chaud, comprenant un dispositif pour la mise en oeuvre du procédé de contrôle décrit ci-dessus.

Selon des formes d'exécution préférées, l'installation de l'invention comporte en outre, en combinaison, une ou plusieurs des caractéristiques suivantes :

- ledit dispositif est situé dans une zone en aval d'une section de bacs de décapage et d'une section de rinçage-séchage de la bande d'acier et en amont d'un accumulateur de sortie ;
- ledit dispositif comprend une caméra thermographique pour mesurer la température apparente de la bande, donc sans application d'une correction d'émissivité, selon un champ de vision comprenant une zone de passage de la bande après passage sur un rouleau déflecteur ;
- l'installation comporte en outre une caméra vidéo, c'est-à-dire opérant dans le domaine visible, dans le même champ de vision que la caméra thermographique précitée.

## Brève description des figures

La figure 1 représente un échantillon de bande d'acier laminée à chaud observé en niveaux de gris par une caméra thermographique avec correction d'émissivité.

La figure 2 représente graphiquement la puissance spécifique rayonnée respectivement par un corps noir, un corps gris et un corps coloré.

La figure 3 représente un échantillon de bande d'acier laminée à chaud observé après séchage par une caméra thermographique sans correction d'émissivité, selon la présente invention.

La figure 4 représente l'échantillon observé comme dans la figure 3 mais après préchauffage dans une étuve jusqu'à 50°C.

La figure 5 représente un échantillon observé comme dans la figure 3, décapé partout sauf en quelques traits larges de quelques millimètres.

La figure 6 représente schématiquement une ligne de décapage industrielle avec sa zone d'implantation d'un capteur selon la présente invention.

Les figures 7 et 8 montrent respectivement une image thermique d'une section de bande d'acier bien décapée sur toute sa largeur ainsi que le profil transversal de température apparente associé, selon la présente invention.

Les figures 9 et 10 montrent respectivement une image thermique d'une section de bande d'acier peu décapée sur toute sa largeur ainsi que le profil transversal de température apparente associé, selon la présente invention.

La figure 11 montre un enregistrement de température apparente maximale en fonction du temps correspondant à une journée de production, sur une ligne de décapage munie d'un système de contrôle selon la présente invention.

La figure 12 montre schématiquement une forme d'exécution alternative selon la présente invention, selon laquelle une caméra opérant dans le domaine visible est montée dans le même champ de vision que le système de mesure thermique précité.

La figure 13 montre le schéma de fonctionnement au cours du temps du système de détection selon la figure 12.

## Description détaillée de l'invention

### Introduction

La présente invention est basée sur la recherche d'une technique de substitution à l'inspection visuelle de la bande en sortie de décapage de manière à détecter le sous-décapage, c'est-à-dire des zones où de l'oxyde subsiste après décapage. En observant la surface de nombreuses bobines en sortie de la ligne de décapage, c'est-à-dire après « décapage + rinçage + séchage », on a constaté que :

-    l'aspect de surface des bandes varie fortement d'une bande à l'autre. Toutes la bandes paraissent « gris métal » mais selon la composition chimique et les paramètres de rinçage, le niveau de gris varie très fortement ;
-    lorsque de l'oxyde subsiste à la surface d'une bande après le décapage, le contraste entre les zones décapées et non décapées demeure faible en terme de niveaux de gris.

Par conséquent, remplacer l'inspection humaine par une inspection automatique suppose non pas de se baser sur un niveau absolu de gris pour détecter les zones d'oxyde restant mais bien d'adapter de manière « intuitive » en fonction de la nuance d'acier, ce qui peut se révéler peu fiable.

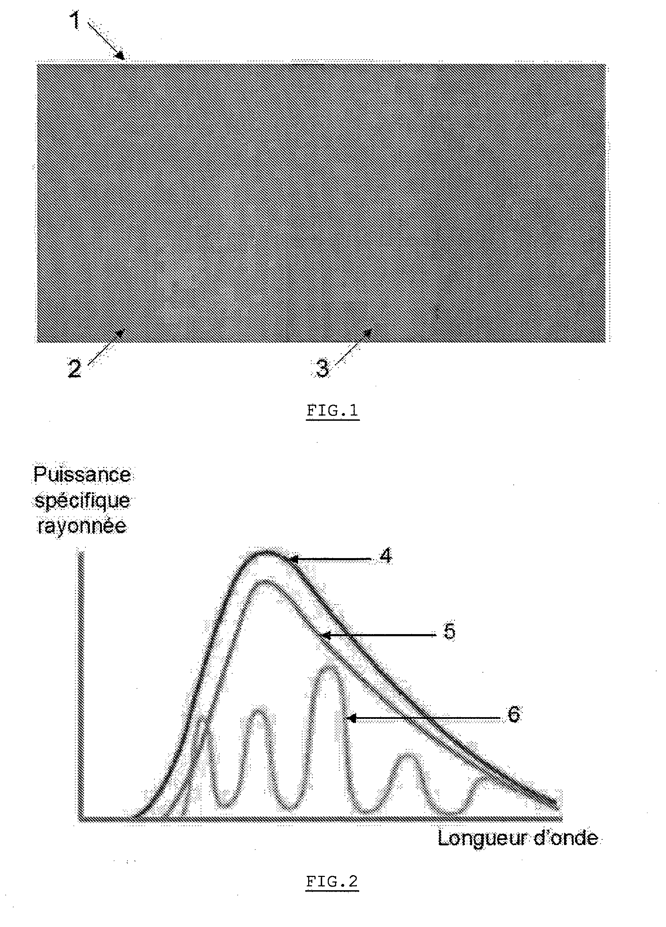

La Fig.1 illustre cette situation. Un échantillon 1 de bande laminée à chaud a été partiellement décapé : dans la zone de gauche 2, l'oxyde a été enlevé par immersion dans une solution acide tandis que dans la zone de droite 3, l'oxyde subsiste. On constate bien que l'écart de gris entre les zones décapée 2 et non décapée 3 reste faible.

### Principe de l'invention

L'invention est basée sur un effet totalement inattendu pour l'homme de métier résultant d'une mesure effectuée de manière, certes inhabituelle, mais qui a permis une détection très claire de zones d'oxyde résiduel.

On a utilisé une mesure faite à l'aide d'une caméra dite thermographique. Les caméras thermographiques permettent d'obtenir une image de la distribution de température sur un objet visé. Celles-ci trouvent des applications de plus en plus nombreuses dans différents domaines :

-    en médecine où elles mettent en évidence les zones superficielles, plus irriguées par le sang ;
-    sur des équipements industriels tels que des armoires électriques ou des composants de distribution électrique de puissance où elles permettent de détecter des surchauffes locales anormales ;
-    en isolation des bâtiments où elles mettent en évidence les fuites d'énergie vers l'extérieur (ponts thermiques).

Les caméras thermographiques sont basées sur le fait que tout corps rayonne une quantité d'énergie qui est fonction de sa température et de son état de surface. Les lois de rayonnement ont été établies pour ce qu'on appelle un « corps noir », c'est-à-dire un corps qui est opaque et ne réfléchit aucun rayonnement. Ce « corps noir » est un radiateur absolu : dans des conditions données, il rayonne le maximum d'énergie. Pour un corps noir, la puissance spécifique rayonnée en fonction de la température et de la longueur d'onde est donnée par la loi de Planck

$$M_\lambda = \frac{2\pi hc^2}{\lambda^5} * \frac{1}{e^{hc/\lambda kT}-1} = \frac{C_1}{\lambda^5} * \frac{1}{e^{C_2/\lambda T}-1},$$

dans laquelle c est la vitesse de la lumière, $C_1 = 3{,}74 \text{ x} \cdot 10^{-16} \text{ W m}^2$, $C_2 = 1{,}44 \text{ x } 10^{-2}$ °K m et h est la constante de Planck.

L'intégrale de la loi de Planck donne la loi de Stefan-Boltzmann :

$$M = \sigma\, T^4 \text{ (en W m}^2\text{)},$$

dans laquelle $\sigma = 5{,}67 \text{ x } 10^{-8} \text{ W m}^{-2}$ °K$^{-4}$.

Cette loi montre que la connaissance de la puissance rayonnée donne accès à la température.

Les corps réels ne rayonnent pas autant d'énergie que ce qui est prédit par les lois ci-dessus ; il faut appliquer un facteur réducteur qu'on appelle « émissivité » et qui représente le rapport entre le rayonnement émis par un corps donné et celui émis par un « corps noir » dans les mêmes conditions. L'émissivité est une fonction de la nature du corps et en particulier de son état de surface. Pour certains corps, l'émissivité est la même quelle que soit la longueur d'onde ; il existe donc un rapport constant entre le rayonnement émis par ce corps et celui émis par un « corps noir » dans les mêmes conditions. Pour d'autres corps, l'émissivité varie en fonction de la longueur d'onde, on appelle ces corps « corps colorés ». Le diagramme présenté sur la Fig.2 illustre cette différence entre les corps : il montre la relation entre la longueur d'onde et la puissance spécifique rayonnée pour trois corps différents :

- le « corps noir » 4 qui obéit à la loi de Planck ;
- le « corps gris » 5 pour lequel la puissance rayonnée est réduite d'un facteur constant quelle que soit la longueur d'onde par rapport au corps noir ;
- le « corps coloré » 6 pour lequel la puissance rayonnée rapportée à celle du « corps noir » varie avec la longueur d'onde.

La mesure de la température d'un corps réel suppose donc d'une part la mesure de la puissance spécifique émise par ce corps et d'autre part la connaissance de son émissivité.

La mesure de la puissance spécifique utilise un système optique compatible avec les longueurs d'ondes mises en jeu et qui focalise le rayonnement sur un détecteur. Différents types de capteurs existent pour transformer la puissance reçue en un signal lumineux qui servira à construire l'image thermique de la cible. De manière non-exhaustive, on peut citer les détecteurs à thermopiles, les détecteurs pyroélectriques et les détecteurs à bolomètres. Ces différents types sont bien connus de l'homme de l'art.

En ce qui concerne la connaissance de l'émissivité qui est aussi indispensable, différentes stratégies ont été développées qui sont aussi bien connues de l'homme de l'art. Elles font l'objet d'une abondante littérature. Elles comprennent entre autres:

- l'étalonnage par un thermocouple ;
- l'application d'un revêtement d'émissivité connue ;
- l'utilisation de pyromètres à plusieurs longueurs d'onde, etc.

Dans tous les cas, il s'agit de mesurer ou de calculer l'émissivité de façon à pouvoir déterminer la température réelle à partir de la « température apparente » c'est-à-dire celle qui supposerait que le corps mesuré est un « corps noir ».

Dans la technique qui fait l'objet de la présente invention, on a utilisé un échantillon partiellement décapé, tel celui de la Fig.1. L'échantillon ayant été séché, on s'est assuré que sa température était homogène puis on en a pris une image à l'aide d'une caméra thermographique réglée comme s'il s'agissait d'un corps noir et on n'en a volontairement pas corrigé les indications (on pourrait également observer la bande point par point à l'aide d'un pyromètre). Il s'agissait donc d'une image de température apparente qui était obtenue. Le résultat est que les zones dans lesquelles subsiste l'oxyde résiduel semblent posséder une température plus élevée que les zones bien décapées et le contraste est, dans ces conditions, très marqué.

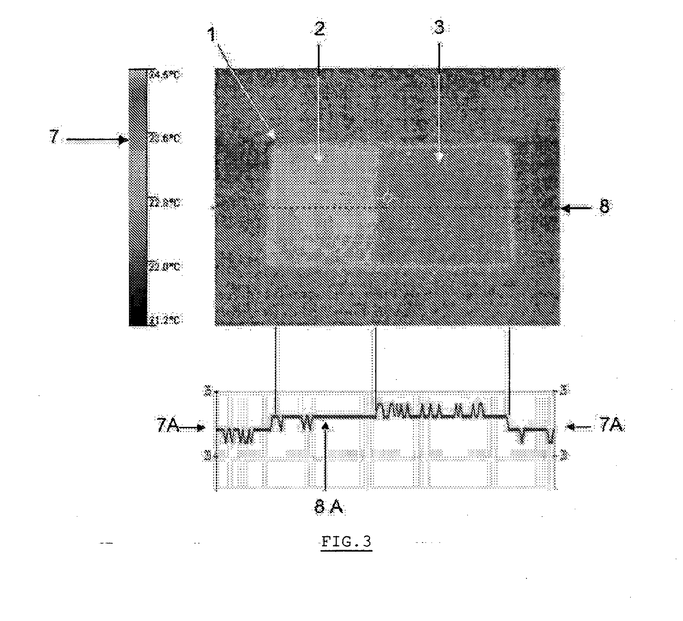

Le résultat est illustré sur la Fig.3. Dans la partie supérieure de la Fig.3, on montre l'image du même échantillon 1 que dans le cas de la Fig.1 : il comprend donc une zone décapée 2 et une zone 3 où l'oxyde subsiste encore. Mais, cette fois, il s'agit d'une image prise à l'aide d'une caméra thermographique, sans aucune correction d'émissivité et qui montre donc la température apparente des différentes zones. Comme l'indique l'échelle de températures 7, codée en couleurs, la température de la zone d'oxyde résiduel 3 apparaît plus élevée que celle de la zone décapée 2, bien que l'échantillon ait en fait une température homogène. Ce résultat est illustré d'une autre façon par le profil de température apparente relevé sur la ligne 8. Le profil de température apparente est présenté sur le diagramme au bas de la Fig.3, avec les échelles repérées 7A ; sur le profil 8A, on observe bien un écart de température apparent d'environ 1°C dans ce cas.

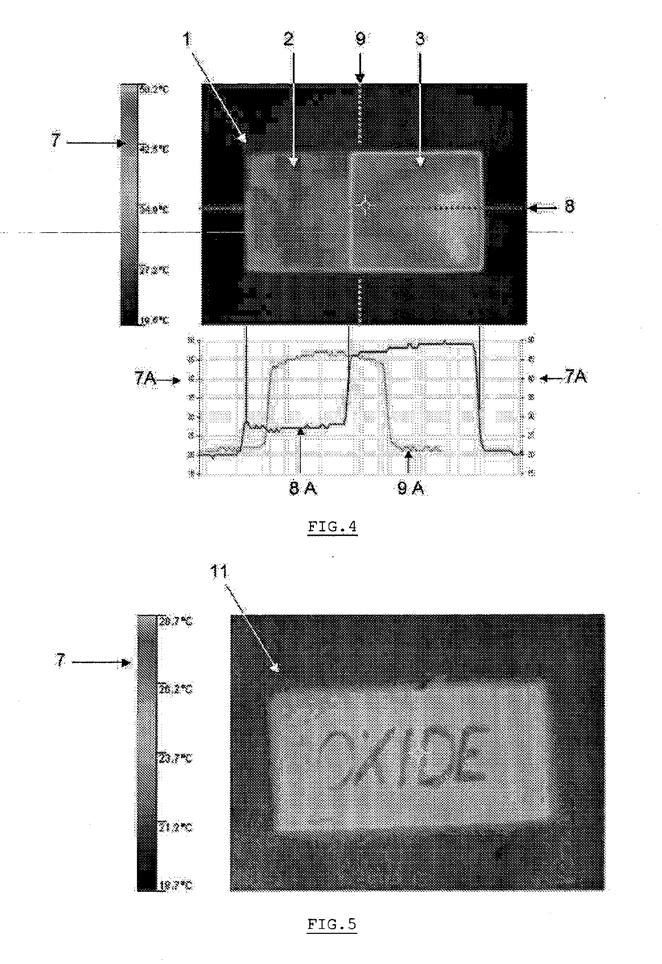

L'effet de la température sur la mesure est illustré par la Fig.4. Cette figure présente les mêmes résultats que la Fig.3 et les repères sont bien entendu identiques. La différence est que, dans le cas de la Fig.4, l'échantillon a été préchauffé dans une étuve, de manière homogène jusqu'à environ 50°C. On constate à nouveau que, comme l'indique l'échelle de températures 7, codée en couleurs, la température de la zone d'oxyde résiduel 3 apparaît plus élevée que celle de la zone décapée 2, bien que l'échantillon ait en fait une température homogène. Ce résultat est illustré d'une autre façon par le profil de température apparente relevé respectivement sur les lignes 8 et 9. Les profils de température apparente sont présentés sur le diagramme au bas de la Fig.4, avec les échelles repérées 7A ; sur le premier profil 8A on observe bien un écart de température apparent qui est maintenant nettement plus élevé puisqu'il se monte à présent à environ 20°C. En outre, le second profil 9A indique que la température apparente est relativement homogène dans la zone 3 d'oxyde résiduel. Il y a donc un gain potentiel à travailler sur une bande à température plus élevée, mais qui doit demeurer homogène.

Un autre test montre avec quelle finesse la technique selon l'invention est capable de détecter des zones d'oxyde résiduel. Un échantillon a été préparé de manière particulière : il a été décapé partout sauf en quelques traits larges de quelques millimètres et qui représentent le mot anglais « oxide ». On a ensuite observé cet échantillon à l'aide d'une caméra thermographique, à nouveau sans effectuer de correction d'émissivité. Le résultat est présenté sur la Fig. 5. On constate que sur l'échantillon 11, les fins traits qui constituent le mot « oxide » apparaissent bien avec une température apparente plus élevée que le fond décapé, comme le montre l'échelle de température 7.

Les tests ont été chaque fois effectués sur une bande sèche de façon à s'affranchir des effets de liquide sur la surface, de façon similaire à celle utilisée dans la demande JP-A-05 070980, déposée par Kawazaki Steel, où on veut contrôler l'efficacité des rouleaux essoreurs par une mesure thermique en aval.

### Description d'une forme d'exécution préférée de l'invention

Le procédé selon l'invention a été appliqué pour contrôler une production industrielle.

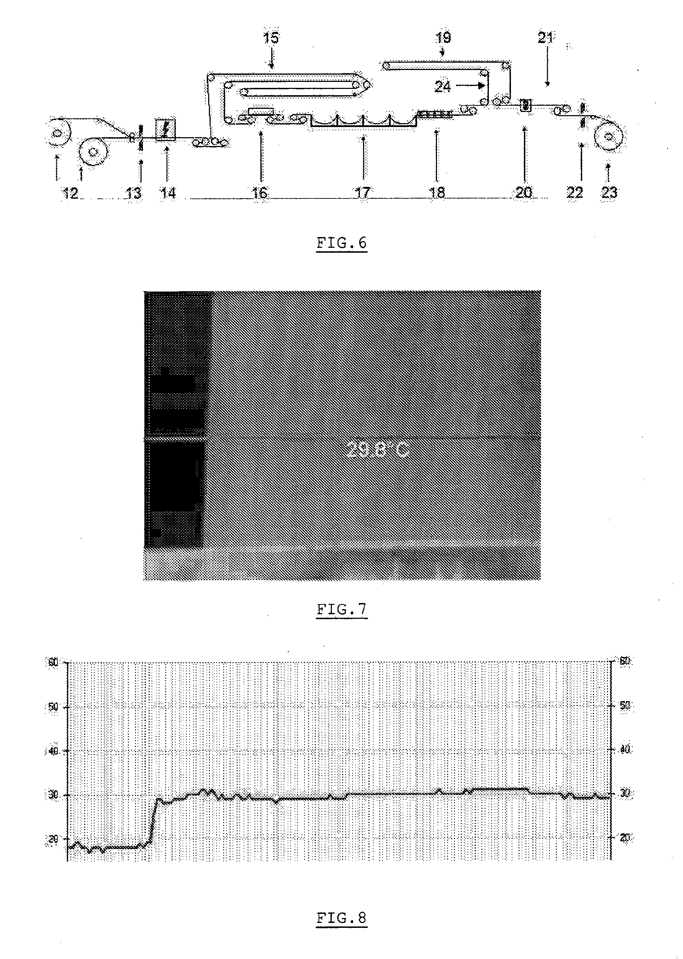

Le schéma de la ligne de décapage et de la zone d'implantation du capteur est présenté sur la Fig. 6. Les composants de la ligne sont les suivants :

- dérouleuses 12 ;
- cisaille 13 ;
- soudeuse 14 pour assembler des bobines et former une bande continue ;
- accumulateur d'entrée 15 ;
- planeuse sous traction 16 pour rendre la bande plus plane et en même temps fissurer l'oxyde par un léger allongement de la bande ;
- bacs de décapage 17 ; dans cet exemple, 4 bacs de décapage au trempé ;
- unité de rinçage et séchage 18 ;
- accumulateur de sortie 19 ;
- cisaille de rives 20 ;
- stand d'inspection 21 ;

- cisaille de sortie 22 ;

- enrouleuse 23.

La zone d'implantation retenue pour le contrôle selon l'invention est la zone située en aval de la section de rinçage-séchage (repère 24) ; la bande y est sèche et généralement à une température d'environ 50°C.
Les résultats de premiers tests sont montrés aux figures suivantes :

- Fig.7 : image thermique d'une section de bande bien décapée ;

- Fig. 8 : profil thermique transversal sur cette même section ;

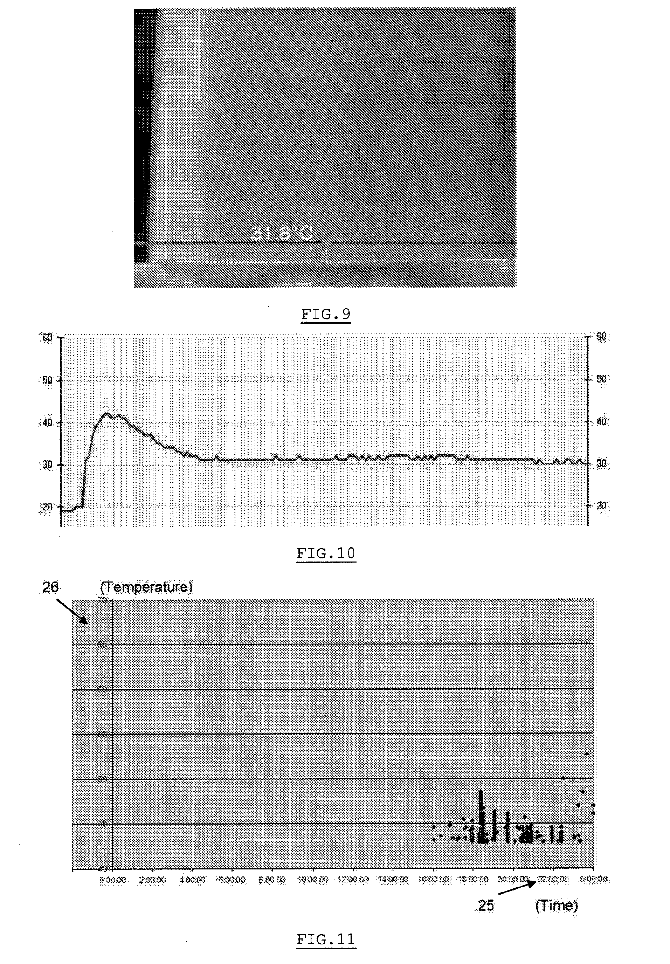

- Fig. 9: image thermique d'une section peu décapée en rive ;

- Fig. 10 : profil thermique en transversal sur cette même section partiellement mal décapée. De manière plus détaillée :

- l'image thermique présentée sur la Fig.7 montre la bande bien décapée qui apparaît à droite de l'image (température basse) ; la bande noire à gauche de l'image représente le fond tandis que la zone située au bas de l'image, plus chaude est l'image d'un rouleau déflecteur ;

- le profil de température apparente présenté sur la Fig.8 est bien un profil plat (avec un creux à gauche correspondant au fond) ;

- l'image thermique présentée sur la Fig.9 montre une section de bande qui n'était pas complètement décapée sur sa rive gauche ; elle apparaît à droite de l'image (température basse) avec à gauche de l'image l'oxyde résiduel qui paraît plus chaud (de nouveau la bande noire à l'extrême gauche représente le fond tandis que la zone au bas de l'image, plus chaude est l'image d'un rouleau déflecteur) ;

- le profil de température apparente présenté sur la Fig.10 est bien un profil plat avec à gauche un plateau d'une température supérieure de 10°C à la moyenne, ce plateau correspondant à la zone mal décapée.

Une autre application dans la même ligne a consisté à remplacer la caméra donnant une image à 2 dimensions par une caméra-ligne qui balaie rapidement la bande transversalement (128 points-image scannés à plus de 500Hz). Un traitement d'image par ordinateur permet ensuite de reconstruire une image 2D qui, en évoluant, permet de suivre ligne par ligne toute la surface de la bande. Une telle disposition est avantageuse dans la mesure où :

- elle permet un balayage plus rapide ;
- donne accès à une image évolutive qui couvre toute la surface de la bande.

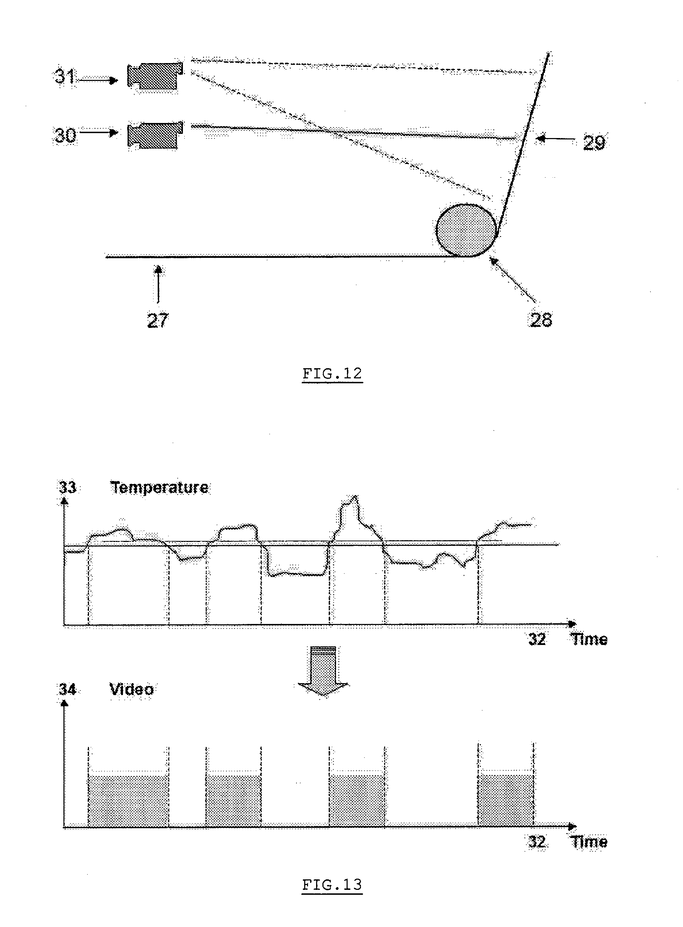

Ce système a été utilisé pour un contrôle de production. La Fig.11 montre un enregistrement correspondant à une journée de production : il s'agit d'un diagramme où la température maximale apparente 26 est représentée en fonction du temps 25.
Une autre application dans la même ligne a consisté à monter une caméra active dans le domaine visible opérant dans le même champ de vision que le système de mesure thermique. Ce montage est présenté sur le schéma de la Fig.12. La bande à contrôler 27, après passage sur un rouleau déflecteur 28 arrive dans la zone d'examen automatique 29. Elle y est observée par un système qui mesure la température apparente 30 et un système vidéo 31 qui vise la même zone. Le schéma de fonctionnement est présenté sur la Fig.13. On voit comment, en fonction du temps 32, évoluent la température apparente 33 et l'action de la caméra vidéo 34. Lorsque des zones d'oxyde résiduel sont détectées par le système thermique, ce qui se traduit par la température apparente 33 franchissant un certain seuil, la caméra vidéo enregistre pendant la période correspondante des images disponibles pour plusieurs usages tels que monitoring des opérateurs de ligne, logging, suivi de qualité, traitement ultérieur, etc.

## Revendications

1.  Procédé de détection d'une couche d'oxyde sur une bande métallique en mouvement dans une installation de fabrication en continu, dans lequel on observe la bande en mouvement à l'aide d'un système de détection sans contact basé sur la mesure du rayonnement thermique émis par ladite bande, **caractérisé en ce qu'**on observe la bande en un endroit où sa température est relativement homogène et **en ce qu'**on mesure la température apparente de la bande en une pluralité de zones, ladite mesure étant effectuée en omettant d'appliquer une correction d'émissivité et **en ce qu'**on identifie une zone recouverte d'oxyde (3) par une valeur de température apparente sensiblement supérieure à celle d'une ou plusieurs autres zones dépourvues d'oxyde (2).

2. Procédé selon la revendication 1, **caractérisé en ce qu'**on fixe le seuil de détection à un écart de température apparente d'au moins 5°C, de préférence au moins 10°C.

3. Procédé selon la revendication 1, **caractérisé en ce qu'**on observe la bande en un endroit où elle est sèche.

4. Procédé selon la revendication 1, **caractérisé en ce qu'**on observe la bande en un endroit où sa température est supérieure à 40°C et de préférence à 50°C.

5. Procédé selon la revendication 1, **caractérisé en ce qu'**on utilise une moyenne mobile des températures apparentes pour appliquer le critère de détection et identifier des zones de dépassement de seuil.

6. Procédé selon la revendication 1, **caractérisé en ce qu'**on observe la bande point par point à l'aide d'un pyromètre.

7. Procédé selon la revendication 1, **caractérisé en ce qu'**on observe toute la surface de la bande en utilisant une caméra thermique.

8. Procédé selon la revendication 1, **caractérisé en ce qu'**on utilise un système de mesure selon une ligne ou une matrice à deux dimensions, de préférence à balayage rapide.

9. Procédé selon la revendication 8, **caractérisé en ce qu'**on acquiert plus de 50 et de préférence 100 points-image par ligne, de préférence sur la largeur de la bande.

10. Procédé selon la revendication 8, **caractérisé en ce que** la fréquence de balayage est supérieure à 100Hz, de préférence supérieure à 500Hz.

11. Procédé selon la revendication 8, **caractérisé en ce qu'**on scrute des zones de l'ordre de 1 cm$^2$.

12. Procédé selon la revendication 8, **caractérisé en ce qu'**on reconstitue une image continue en juxtaposant les lignes par traitement d'image.

13. Procédé selon la revendication 1, **caractérisé en ce que** l'on couple la détection thermique avec une détection vidéo fonctionnant essentiellement dans la même zone de scrutation.

14. Procédé selon la revendication 13, caractérisé ne ce que le système vidéo est activé lorsque la température apparente dépasse un certain seuil.

15. Procédé selon la revendication 14, **caractérisé en ce que** le signal vidéo est utilisé comme alarme et indication de zone à défaut pour un opérateur, comme enregistrement pour le contrôle qualité ou en vue d'un traitement ultérieur.

16. Procédé selon l'une quelconque des revendications 1 à 15, **caractérisé en ce qu'**il est appliqué pour contrôler le décapage de bandes d'acier laminées à chaud, en détectant les zones d'oxyde résiduel qui subsistent après décapage.

17. Procédé selon l'une quelconque des revendications 1 à 15, **caractérisé en ce qu'**il est appliqué pour détecter un oxyde résiduel sur bande d'acier laminée à chaud, après décalaminage.

18. Procédé selon l'une quelconque des revendications 1 à 15, **caractérisé en ce qu'**il est appliqué pour détecter la présence d'oxyde sur des cylindres de laminage.

19. Installation de décapage intégrée dans une ligne de production de bandes d'acier, de préférence laminées à chaud, **caractérisée en ce qu'**elle comprend un dispositif (24) pour la mise en oeuvre du procédé selon l'une quelconque des revendications 1 à 15.

**20.** Installation selon la revendication 19, **caractérisée en ce que** ledit dispositif est situé dans une zone en aval d'une section de bacs de décapage (17) et d'une section de rinçage-séchage (18) de la bande d'acier et en amont d'un accumulateur de sortie (19).

**21.** Installation selon la revendication 19 ou 20, **caractérisée en ce que** ledit dispositif (24) comprend une caméra thermographique (30) pour mesurer la température apparente de la bande, donc sans application d'une correction d'émissivité, selon un champ de vision comprenant une zone de passage de la bande après passage sur un rouleau déflecteur (28).

**22.** Installation selon la revendication 21, **caractérisée en ce qu'**elle comporte en outre une caméra vidéo (31), c'est-à-dire opérant dans le domaine visible, dans le même champ de vision que la caméra thermographique précitée (30).


**Patentansprüche**

**1.** Detektionsverfahren einer Oxidschicht auf einem Metallband in Bewegung in einer kontinuierlichen Fertigungsanlage, bei dem das Band in Bewegung mit Hilfe eines kontaktlosen Detektionssystems, das auf der Messung der von dem Band gesendeten thermischen Strahlung basiert, beobachtet wird, **dadurch gekennzeichnet, dass** das Band an einer Stelle beobachtet wird, an der seine Temperatur relativ gleich ist und dass die scheinbare Temperatur des Bands in einer Vielzahl von Bereichen gemessen wird, wobei die Messung bei Auslassung der Anwendung einer Emissivitätskorrektur durchgeführt wird und dass ein mit Oxid bedeckter Bereich (3) durch einen Wert der scheinbaren Temperatur festgestellt wird, der deutlich höher als der eines oder mehrerer anderer Bereiche ohne Oxid (2) ist.

**2.** Verfahren nach Anspruch 1, **dadurch gekennzeichnet, dass** der Detektionsgrenzwert auf eine Differenz der scheinbaren Temperatur von mindestens 5 °C, vorzugsweise von mindestens 10 °C, festgelegt ist.

**3.** Verfahren nach Anspruch 1, **dadurch gekennzeichnet, dass** das Band an einer Stelle beobachtet wird, an der es trocken ist.

**4.** Verfahren nach Anspruch 1, **dadurch gekennzeichnet, dass** das Band an einer Stelle beobachtet wird, an der seine Temperatur höher als 40 °C und vorzugsweise höher als 50 °C ist.

**5.** Verfahren nach Anspruch 1, **dadurch gekennzeichnet, dass** ein mobiler Durchschnitt der scheinbaren Temperaturen verwendet wird, um das Detektionskriterium anzuwenden und Bereiche festzustellen, in denen der Grenzwert überschritten wird.

**6.** Verfahren nach Anspruch 1, **dadurch gekennzeichnet, dass** das Band Punkt für Punkt mit Hilfe eines Pyrometers beobachtet wird.

**7.** Verfahren nach Anspruch 1, **dadurch gekennzeichnet, dass** die gesamte Oberfläche des Bands mit einer thermischen Kamera beobachtet wird.

**8.** Verfahren nach Anspruch 1, **dadurch gekennzeichnet, dass** ein Messsystem gemäß einer Linie oder einer zweidimensionalen Matrix, vorzugsweise mit Schnellabtastung, verwendet wird.

**9.** Verfahren nach Anspruch 8, **dadurch gekennzeichnet, dass** mehr als 50 und vorzugsweise 100 Bildpunkte je Linie, vorzugsweise über die Breite des Bands, erfasst werden.

**10.** Verfahren nach Anspruch 8, **dadurch gekennzeichnet, dass** die Abtastfrequenz höher als 100 Hz, vorzugsweise höher als 500 Hz, ist.

**11.** Verfahren nach Anspruch 8, **dadurch gekennzeichnet, dass** Bereiche von 1 cm$^2$ abgesucht werden.

**12.** Verfahren nach Anspruch 8, **dadurch gekennzeichnet, dass** durch Nebeneinanderlegen der Linien durch Bildbearbeitung ein kontinuierliches Bild wiederhergestellt wird.

**13.** Verfahren nach Anspruch 1, **dadurch gekennzeichnet, dass** die thermische Detektion mit einer Videodetektion gekoppelt ist, die im Wesentlichen im selben Suchbereich funktioniert.

14. Verfahren nach Anspruch 13, **dadurch gekennzeichnet, dass** das Videosystem aktiviert ist, wenn die scheinbare Temperatur einen bestimmten Grenzwert überschreitet.

15. Verfahren nach Anspruch 14, **dadurch gekennzeichnet, dass** das Videosignal als Alarm und Anzeige eines fehlerhaften Bereichs für einen Techniker, als Registrierung für die Qualitätskontrolle oder im Hinblick auf eine spätere Behandlung verwendet wird.

16. Verfahren nach einem der Ansprüche 1 bis 15, **dadurch gekennzeichnet, dass** es angewendet wird, um das Abbeizen von warmgewalzten Stahlbändern durch Detektion der restlichen Oxidbereiche, die nach dem Abbeizen verbleiben, zu kontrollieren.

17. Verfahren nach einem der Ansprüche 1 bis 15, **dadurch gekennzeichnet, dass** es angewendet wird, um nach dem Entzundern ein Restoxid auf warmgewalztem Stahlblech festzustellen.

18. Verfahren nach einem der Ansprüche 1 bis 15, **dadurch gekennzeichnet, dass** es angewendet wird, um die Anwesenheit von Oxid auf Walzzylindern festzustellen.

19. Abbeizanlage, die in eine Produktionslinie von vorzugsweise warmgewalzten Stahlbändern integriert ist, **dadurch gekennzeichnet, dass** sie eine Vorrichtung (24) für die Umsetzung des Verfahrens nach einem der Ansprüche 1 bis 15 umfasst.

20. Anlage nach Anspruch 19, **dadurch gekennzeichnet, dass** sich die Vorrichtung in einem Bereich nach einem Abschnitt mit Abbeizbehältern (17) und einem Spül- und Trocknungsabschnitt (18) des Stahlbands und vor einem Ausgangsspeicher (19) befindet.

21. Anlage nach Anspruch 19 oder 20, **dadurch gekennzeichnet, dass** die Vorrichtung (24) eine thermografische Kamera (30) umfasst, um die scheinbare Temperatur des Bands zu messen, also ohne Anwendung einer Emissivitätskontrolle, gemäß einem Sichtfeld, das nach Passieren einer Deflektorwalze (28) einen Übergangsbereich des Bands umfasst.

22. Anlage nach Anspruch 21, **dadurch gekennzeichnet, dass** sie ferner eine Videokamera (31) aufweist, das heißt, die im sichtbaren Bereich im selben Sichtfeld wie die vorgenannte thermografische Kamera (30) arbeitet.

**Claims**

1. A method for detecting an oxide layer on a metal strip in motion in a continuous manufacturing facility, in which the strip in motion is observed by means of a contactless detection system based on the measurement of the thermal radiation emitted by said strip, **characterized in that** the strip is observed in a location where its temperature is relatively homogeneous, **in that** the apparent temperature of the strip is measured in a plurality of areas, said measurement being carried out by omitting to apply any emissivity correction, and **in that** an area covered with oxide (3) is identified by an apparent temperature value that is substantially greater than that of one or several oxide-free (2) areas.

2. The method according to Claim 1, **characterized in that** the detection threshold is set to an apparent temperature deviation of at least 5°C, preferably at least 10°C.

3. The method according to Claim 1, **characterized in that** the strip is observed in a location where it is dry.

4. The method according to Claim 1, **characterized in that** the strip is observed in a location where its temperature is above 40°C, and preferably above 50°C.

5. The method according to Claim 1, **characterized in that** a mobile average of the apparent temperatures is used for applying the detection criterion and identifying areas where the threshold is exceeded.

6. The method according to Claim 1, **characterized in that** the strip is observed point by point by means of a pyrometer.

7. The method according to Claim 1, **characterized in that** the whole surface of the strip is observed by using a thermal camera.

8. The method according to Claim 1, **characterized in that** a measurement system along a line or a two-dimensional matrix, preferably with rapid sweeping, is used.

9. The method according to Claim 8, **characterized in that** more than 50 and preferably 100 image-points are acquired per line, preferably over the width of the strip.

10. The method according to Claim 8, **characterized in that** the sweeping frequency is greater than 100 Hz, preferably greater than 500 Hz.

11. The method according to Claim 8, **characterized in that** areas of the order of 1 cm$^2$ are examined.

12. The method according to Claim 8, **characterized in that** a continuous image is reconstructed by juxtaposing the lines by image processing.

13. The method according to Claim 1, **characterized in that** thermal detection is coupled with video detection essentially operating in the same examined area.

14. The method according to Claim 13, **characterized in that** the video system is activated when the apparent temperature exceeds a certain threshold.

15. The method according to Claim 14, **characterized in that** the video signal is used as an alarm and as the indicator of a faulty area for an operator, as well as used as a record for quality control or with a view to subsequent processing.

16. The method according to any of Claims 1 to 15, **characterized in that** it is applied for controlling the stripping of hot-rolled steel strips, by detecting the areas of residual oxide that subsist after stripping.

17. The method according to any of Claims 1 to 15, **characterized in that** it is applied for detecting a residual oxide on a hot-rolled steel strip, after scale removal.

18. The method according to any of Claims 1 to 15, **characterized in that** it is applied for detecting the presence of oxide on rolling cylinders.

19. A stripping installation integrated into a production line of steel strips, preferably hot-rolled, **characterized in that** it comprises a device (24) for implementing the method according to any of Claims 1 to 15.

20. The installation according to Claim 19, **characterized in that** said device is located in an area downstream from a section of stripping baths (17) and from a rinsing-drying section (18) for the steel strip and upstream from an output accumulator (19).

21. The installation according to Claim 19 or 20, **characterized in that** said device (24) comprises a thermographic camera (30) for measuring the apparent temperature of the strip, therefore without applying any emissivity correction, according to a field of view comprising an area where the strip passes after passing over a deflecting roller (28).

22. The installation according to Claim 21, **characterized in that** it further comprises a video camera (31), i.e. operating in the visible range, in the same field of view as the aforementioned thermographic camera (30).

EP 2 235 474 B1

FIG.1

FIG.2

FIG.3

FIG.4

FIG.5

FIG.6

FIG.7

FIG.8

FIG.9

FIG.10

26 (Temperature)

25 (Time)

FIG.11

FIG.12

FIG.13

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- JP 2003231981 A
- JP 11325839 A
- JP 11189885 A
- JP 10306391 A
- DE 10332693 A
- EP 1162287 A
- JP 5070980 A